# FASCICULE DE BREVET EUROPEEN

(11) **EP 1 684 731 B1**
(45) Date de publication et mention de la délivrance du brevet: **04.07.2012**
(21) Numéro de dépôt: 04786324.6
(22) Date de dépôt: 18.08.2004
(51) Int. Cl.: A61K 9/28, C09D 103/00, C09D 103/02

(54) **COMPOSITION AMYLACEE FILMOGENE**
FILMBILDENDE STÄRKEHALTIGE ZUSAMMENSETZUNG
FILM-FORMING STARCHY COMPOSITION

(30) Priorité: 20.11.2003 FR 0313604
(43) Date de publication de la demande: 02.08.2006
(73) Titulaire: ROQUETTE FRERES, 62136 Lestrem (FR)
(72) Inventeur: LEFEVRE, Philippe, F-59660 Haverskerque (FR); FRANCOIS, Alain, F-62131 Drouvin Le Marais (FR); FACON, Philippe, F-59940 Estaires (FR); QUETTIER, Claude, FR-59130 Lambersart (FR); PARISSAUX, Xavier, F-62510 Arques (FR)
(74) Mandataire: Touati, Catherine
(86) Numéro de dépôt international: PCT/FR2004/002158
(87) Numéro de publication internationale: WO 2005/060944

(56) Documents cités:
- EP-A- 0 735 080
- EP-A- 0 945 487
- EP-A- 1 245 577
- WO-A-00/36006
- WO-A-01/92400
- WO-A-02/00205
- WO-A-90/13576
- WO-A-99/21536
- US-A- 5 498 706

## Description

L'invention a pour objet une composition filmogène à base d'amidon ainsi qu'un procédé de pelliculage de formes solides pharmaceutiques, alimentaires, agricoles, et de préparation de films mettant en oeuvre ladite composition.

On entend par formes solides au sens de la présente invention toute présentation de substances alimentaires, pharmaceutiques, cosmétiques, chimiques ou agrochimiques, sous forme par exemple de comprimés, gélules, capsules, pellets, microsphères, granulés, semences, graines, cristaux ou poudres, etc...

Les amidons natifs et modifiés sont traditionnellement utilisés dans l'industrie pharmaceutique notamment en tant que diluant, agent désintégrant, liant, pour la préparation de comprimés, le remplissage de gélules. Les amidons natifs sont rarement utilisés pour le pelliculage, en raison principalement de leur caractère insoluble dans l'eau froide, ce qui nécessite obligatoirement une précuisson préalable de l'amidon afin de le solubiliser.

Le pelliculage de formes solides, en particulier de comprimés, est une opération fréquente qui vise à obtenir une protection physique et chimique du principe actif. Le médicament est ainsi protégé de son environnement (humidité, oxygène, lumière). Le pelliculage permet également de masquer le goût, l'odeur ou la couleur de ce principe actif, et permet aussi de modifier sa libération dans l'organisme en augmentant la résistance du comprimé aux sucs gastriques. Le pelliculage facilite l'ingestion des comprimés, améliore leur apparence et leur intégrité mécanique. La plupart des formes solides peuvent être pelliculées : comprimés, gélules, capsules, granulés.

Le pelliculage consiste en l'application d'une composition liquide filmogène par exemple sur des comprimés, cette composition devenant après séchage un film protecteur. L'agent filmogène idéal présente de préférence un poids moléculaire élevé, une viscosité faible, et une bonne adhésion au support. Il doit permettre l'obtention d'un film élastique, cohésif, adhérant à la forme qu'il revêt. Il est de préférence soluble dans l'eau, l'eau étant le solvant préféré par rapport aux solvants organiques en raison de sa facilité d'utilisation.

Il existe sur le marché bon nombre de compositions filmogènes à base de polymères de cellulose, d'acrylate. On peut citer en particulier l'hydroxypropyl méthylcellulose (HPMC) couramment utilisée pour le pelliculage de comprimés. L'on peut rencontrer certaines difficultés en utilisant ce type de polymères, telles que des problèmes d'adhérence aux supports, de craquage ou fissurage de l'enrobage, ou des phénomènes de remplissage des gravures souvent présentes sur les comprimés (logos, dosages). De plus, l'HPMC présente l'inconvénient d'avoir un goût et/ou une odeur désagréable. Les polymères synthétiques sont en outre commercialisés à des prix assez élevés.

L'amidon est depuis longtemps utilisé en tant qu'agent filmogène dans l'industrie textile ou papetière. Les principales sources industrielles d'amidon sont, par ordre d'importance décroissante, le mais, le blé, la pomme de terre, le manioc et la patate douce. En fonction de son origine, l'amidon présente des variations de composition, en particulier en ce qui concerne les pourcentages d'amylose et d'amylopectine. L'amylopectine est la composante ramifiée de l'amidon où les unités d'α-D glucose sont polymérisées par des liaisons αD(1-4) et sont reliées avec des branchements en αD(1-6).

L'amylose est la composante linéaire de l'amidon, qui ne renferme pratiquement que des liaisons αD(1-4).

Certaines variétés d'amidon, dites « waxy » sont constituées essentiellement d'amylopectine. D'autres, dites « riches en amylose » sont constituées de plus de 50% et généralement de 70 à 75% d'amylose.

Les amidons riches en amylose sont connus depuis longtemps pour leurs propriétés filmogènes et ont toujours semblé supérieurs aux autres amidons dans cette application. Ils présentent toutefois l'inconvénient de nécessiter une mise en oeuvre à haute température, c'est à dire environ 80°C pour éviter que ces amidons ne rétrogradent. Ceci est particulièrement gênant pour l'enrobage de formes solides puisque les équipements usuels de pelliculage ne sont pas prévus pour travailler à de telles températures. De plus, il faut veiller à ce qu'aucun point du circuit ne soit froid, ce qui occasionnerait une gélification de la composition dans les circuits.

Les amidons riches en amylopectine ne sont pas utilisables en particulier dans les procédés de pelliculage en turbine, étant donné qu'on observe lors de leur utilisation une agglomération des centres à pelliculer entre-eux. Ce défaut rédhibitoire est très difficile à corriger, engendre un enrobage final hétérogène et incomplet, et impose des formulations complexes. Les amidons waxy sont en outre très visqueux en solution, ce qui explique probablement les difficultés de mise en oeuvre qu'ils engendrent en pelliculage.

Le brevet WO 02/00205 décrit l'utilisation d'amidon riche en amylose acétylé et prégélatinisé pour l'enrobage de comprimés, la fabrication de capsules et de « caplets » (comprimés enrobés d'un film coloré épais ressemblant à une gélule). L'amidon comprend plus de 50% d'amylose et est de préférence issu du mais.

Le brevet EP 1.245.577 dont la Demanderesse est titulaire décrit un procédé de préparation d'amidons riches en amylose prégélatinisés, et leurs applications en pelliculage, capsules molles, gélules, films rafraîchissants.

Le brevet WO 02/092708 décrit un procédé de préparation d'une composition filmogène, consistant en l'extraction en milieu alcoolique de l'amylose d'un amidon en comprenant plus de 50%. La composition filmogène comprend donc de l'amylose pure, et peut comprendre en outre un agent plastifiant.

Le brevet EP 945.487 décrit des films à base de dérivés d'amidon ou d'amylose, fabriqués à partir d'une solution d'éther hydroxypropylique d'amidon, la teneur en amylose de la matière brute initiale étant supérieure à 60%. En particulier, un amidon de pois ridé, comprenant environ 75% d'amylose, est utilisé.

Le brevet EP 1.024.795 décrit des mélanges d'amylose et d'éthylcellulose, l'amylose seule n'étant pas stable par rapport à la reprise en eau.

WO 00/36006 décrit une composition contenant un amidon (amylose) modifié par une réaction d'hydroxyalkylation, un autre amidon qui peut être issu du pois, un polymère filmogène (alcool polyvinylique ou acétate de polyvinyle), un plastifiant polyhydroxylé, un acide gras et éventuellement de l'eau. Cette composition est utilisée pour la fabrication de feuilles et de films flexibles et biodégradables.

US 5.498.706 décrit une composition comprenant de l'amidon de pois particulier ayant une teneur en amylose égale ou supérieure à 70% modifié par acétylation et du citrate d'éthyle comme agent plastifiant pour former une feuille transparente et flexible.

Bien que les propriétés filmogènes des amidons riches en amylose soient intéressantes, leur mise en oeuvre reste assez contraignante en pratique, en raison de leur rétrogradation rapide au refroidissement. Ceci peut être évité en effectuant une pulvérisation à chaud de la solution filmogène, mais il existe alors un risque de bouchage des busses dû à la rétrogradation de l'amidon pendant la pulvérisation. Les solutions de pelliculage à base de polymères synthétiques sont actuellement préparées et pulvérisées à température ambiante. Il serait donc très intéressant et beaucoup plus pratique en industrie de pouvoir disposer d'un amidon filmogène qui puisse être mis en oeuvre à une température de travail la plus proche possible de la température ambiante, tout en restant stable dans ces conditions. On cherche également à pouvoir fabriquer un film ou un pelliculage de forme solide qui soit imperméable à l'eau, permettant de formuler des enrobages barrière à l'eau ou à dissolution retardée. De même, le formulateur recherche pour diminuer le temps de pelliculage, la matière sèche la plus élevée possible et une viscosité compatible avec la pulvérisation par buse, ce qui peut poser problème lorsqu'on travaille avec des amidons riches en amylose, développant une forte viscosité en solution. L'idéal serait de pouvoir disposer d'une solution d'enrobage de matière sèche supérieure à 20% et de préférence voisine de 30%, présentant une viscosité inférieure à 500 mPa.s à la température de travail. Ceci permettrait de s'affranchir de toute étape de chauffage et de maintien des solutions à chaud.

Il n'existe donc pas à l'heure actuelle de solution satisfaisante pour réaliser des films ou des pelliculages satisfaisants à partir d'amidons comme seul agent filmogène, afin de pouvoir remplacer les polymères synthétiques assez coûteux.

Cherchant à pallier ce manque, la Demanderesse a alors réalisé de nombreux travaux visant à remplacer tout ou partie des polymères synthétiques par une matière amylacée ne présentant pas les inconvénients des amidons précités.

Et elle a eu le mérite de trouver que ce but pouvait être atteint dès lors qu'on met en oeuvre pour préparer une composition filmogène un amidon ou un mélange d'amidons dont la teneur en amylose est sélectionnée dans une plage particulière, comprise entre 25 et 45%,

et comprenant au moins un amidon de légumineuse hydroxypropylé ou acétylé présentant une teneur en amylose inférieure à 45%.

La présente invention a donc pour objet une composition amylacée filmogène pour le pelliculage de formes solides ou la préparation de films caractérisée en ce qu'elle présente une teneur en amylose comprise entre 25 et 45%, de préférence comprise entre 30 et 44%, encore plus préférentiellement comprise entre 35 et 40%, et mieux entre 35 et 38% ces pourcentages étant exprimés en poids sec par rapport au poids sec d'amidon contenu dans ladite composition, et qu'elle comprend au moins un amidon de légumineuse hydroxypropylé ou acétyle présentant une teneur en amylose inférieure à 45%.

Conformément à la présente invention, cet amidon de légumineuse présente une teneur en amylose inférieure à 45%, plus précisément comprise entre 25 et 45%, de préférence comprise entre 30 et 44%, et encore plus préférentiellement comprise entre 35 et 40%. Par « légumineuse » au sens de la présente invention, on entend toute plante appartenant aux familles des césalpiniacées, des mimosacées ou des papilionacées et notamment toute plante appartenant à la famille des papilionacées comme, par exemple, le pois, le haricot, la fève, la fèverole, la lentille, la luzerne, le trèfle ou le lupin.

Cette définition inclut notamment toutes les plantes décrites dans l'un quelconque des tableaux contenus dans l'article de R. HOOVER et al. intitulé « Composition, structure, functionality and chemical modification of legume starches : a review ».

De préférence, la légumineuse est choisie dans le groupe comprenant le pois, le haricot, la fève et la fèverole.

Avantageusement, il s'agit de pois, le terme « pois » étant ici considéré dans son acception la plus large et incluant en particulier :
- toutes les variétés sauvages de « pois lisse » (« smooth pea »), et
- toutes les variétés mutantes de « pois lisse » et de « pois ridé » (« wrinkled pea ») et ce, quelles que soient les utilisations auxquelles on destine généralement lesdites variétés (alimentation humaine, nutrition animale et/ou autres utilisations).

Lesdites variétés mutantes sont notamment celles dénommées « mutants r », « mutants rb », « mutants rug 3 », « mutants rug 4 », « mutants rug 5 » et « mutants lam » tels que décrits dans l'article de C-L HEYDLEY et al. intitulé « Developing novel pea starches » Proceedings of the Symposium of the Industrial Biochemistry and Biotechnology Group of the Biochemical Society, 1996, pp. 77-87.

Selon une autre variante avantageuse, la légumineuse est une plante, par exemple une variété de pois ou de féverole, donnant des graines contenant au moins 25 %, de préférence au moins 40 %, en poids d'amidon (sec/sec).

Par « amidon de légumineuse », on entend toute composition extraite et ce, de quelque manière que ce soit, d'une légumineuse et notamment d'une papilionacée, et dont la teneur en amidon est supérieure à 40 %, de préférence supérieure à 50 % et encore plus préférentiellement supérieure à 75 %, ces pourcentages étant exprimés en poids sec par rapport aux poids sec de ladite composition.

Avantageusement, cette teneur en amidon est supérieure à 90 % (sec/sec). Elle peut en particulier être supérieure à 95 %, y compris supérieure à 98 %.

Selon une autre variante, la teneur en protéines de ladite composition est inférieure à 25 %, de préférence inférieure à 10 %, ces pourcentages étant exprimés en poids sec par rapport au poids sec de ladite composition. Cette teneur peut notamment être inférieure à 5 %, y compris inférieure à 1 %.

La teneur en amidon de la composition filmogène conforme à l'invention est comprise entre 10 et 90% en poids, de préférence entre 10 et 50%, et plus préférentiellement encore entre 10 et 30%.

L'amidon contenu dans ladite composition a subi au moins un traitement de modification choisi dans le groupe comprenant les traitements chimiques.

Selon l'invention, les traitements chimiques convenant à l'obtention d'une composition filmogène sont les traitements dits de stabilisation que sont l'hydroxypropylation, l'acétylation, ces traitements pouvant éventuellement être complétés par une fluidification, par exemple par traitement acide. On obtient dans ce cas une composition filmogène qui présente des propriétés tout à fait semblables au polymère synthétique de référence : l'hydroxypropyl méthylcellulose. La composition selon l'invention comprend donc avantageusement au moins un amidon stabilisé, et de préférence un amidon hydroxypropylé présentant un degré de substitution (DS) de 0,2 maximum. On entend par DS au sens de la présente invention le nombre moyen de groupements hydroxypropyl pour 10 unités d'anhydroglucose. Ce nombre moyen est déterminé par les méthodes analytiques usuelles très connues de l'homme du métier.

La composition filmogène conforme à l'invention peut en outre comprendre un ou plusieurs agents filmogènes secondaires, qui peuvent être choisis parmi les dérivés cellulosiques tels que les alkyl éthers ou alkyl esters, comme par exemple méthylcellulose, carboxyméthylcellulose (CMC), hydroxypropyl cellulose (HPC), hydroxypropyl méthylcellulose, acétophtalate de cellulose, acétate de cellulose ou éthylcellulose, ou encore la polyvinylpyrrolidone (PVP), le phtalate de polyvinyle, le dextrose, la zéine, le pullulan, les polymères acryliques, les alginates, les carraghénates, le polyvinyl alcool (PVA), la gélatine, les dextrines, et leurs mélanges. Cet agent filmogène secondaire a un rôle renforçateur de couverture, et permet d'éviter d'éventuelles fissurations du revêtement créé y compris son altération lors des manipulations ultérieures.

Avantageusement, on utilisera une hydroxypropyl méthylcellulose, de faible viscosité (comprise entre 3 et 15 centipoises à température ambiante, en solution à 2% en poids dans l'eau). Selon une variante préférée, on utilisera un mélange d'hydroxyéthyl cellulose, d'hydroxypropyl méthylcellulose et d'amidon conforme à l'invention, le rapport pondéral entre l'hydroxyéthyl cellulose et l'hydroxypropyl méthylcellulose étant avantageusement compris entre 1:4 et 1:1, de préférence entre 1:3 et 1:2.

L'agent filmogène secondaire sera présent dans la composition filmogène conforme à l'invention à hauteur de 0 à 55% en poids sur sec, de préférence de 3,5 à 50%, et encore plus préférentiellement de 5 à 20%.

La composition filmogène conforme à l'invention peut avantageusement comprendre un ou plusieurs agents plastifiants, hydrophiles ou hydrophobes. Cet agent plastifiant peut être choisi dans le groupe constitué notamment par le glycérol, le sorbitol et les anhydrides de sorbitol, le maltitol et les sirops de maltitol, le polyéthylène glycol de poids moléculaire compris entre 400 et 10000 daltons et le stéarate de polyéthylène glycol, le propylène glycol, le triéthylcitrate, l'acétyltriéthylcitrate, tributylcitrate, le polysorbate, les monoglycérides acétylés, les esters d'acide lactique, les acides gras et leurs sels ou dérivés éthoxylés comme notamment l'acide stéarique, les phtalates, les sébaçates d'éthyle ou de butyle, le miglyol, le glycérol triacétate, la paraffine liquide, la lécithine, la cire de Carnauba, l'huile de ricin hydrogénée seuls ou en mélange entre eux. Un agent plastifiant préféré est l'acide stéarique éthoxylé ou la triacétine.

Des teneurs en plastifiant comprises entre 2,5 et 30% conviennent bien, les pourcentages étant exprimés par rapport à l'amidon contenu dans la composition filmogène. Selon une variante préférée, la composition filmogène selon l'invention comprend, entre 5 et 15% et encore plus préférentiellement entre 7,5 et 10 % d'agent plastifiant en poids sec par rapport à la composition filmogène.

La composition filmogène peut également comprendre tout additif approprié couramment utilisé par l'homme de l'art tel que des arômes, des agents édulcorants, des colorants ou pigments, des opacifiants tels que le talc, des lubrifiants tels que le stéarate de magnésium, les huiles minérales, la lécithine, la cire de Carnauba, des agents imperméabilisants tels que les acides gras et leurs dérivés, les polymères de silicone, des agents mouillants tels que des tensio-actifs, des surfactants tels que le polysorbate 80, des agents améliorant l'adhésion du film tels que la cellulose microcristalline, les polyols, maltodextrines, polydextrose ou le lactose, des conservateurs tels que notamment le citrate de sodium, des agents anti-collant comme le polyéthylène glycol 3350, la lécithine, l'acide stéarique, le talc, la cellulose microcristalline, des solvants aqueux tels que méthanol, méthanol, butanol, chlorure de méthylène, acétone ainsi que des substances actives par exemple pharmaceutiques.

Les pigments de coloration susceptibles d'être utilisés peuvent être choisis parmi les pigments utilisés jusqu'alors dans la fabrication de produits filmogènes destinés au revêtement de formes solides pharmaceutiques ou alimentaires. On pourra utiliser tout pigment ou colorant solubles ou sous forme de laques, de qualité alimentaire ou pharmaceutique, et notamment le dioxyde de titane, le talc, le carbonate de magnésium, -les oxydes de fer, la riboflavine. Les teneurs en colorant varient suivant le type de coloration recherché : pour les revêtements blancs, on préférera utiliser 20 à 50% en poids de dioxyde de titane ; pour les revêtements colorés, on utilisera 0,1 à 40%, de préférence 15 à 25% en poids de colorant.

Les agents lubrifiants peuvent être utilisés à raison de 0 à 10% en poids dans la composition filmogène. Les conservateurs sont généralement utilisés à raison de 0 à 4% en poids. Les surfactants sont incorporés à raison de 0 à 15% en poids.

Elle peut comprendre en outre de l'eau, et généralement des teneurs en eau comprises entre 10 et 90% en poids. De préférence, ladite composition comprend de 60 à 85% en poids d'eau, et encore plus préférentiellement de 70 à 80% d'eau.

La composition filmogène conforme à l'invention peut également se présenter sous forme de poudre prête à l'emploi. Elle peut consister soit en un mélange physique de poudres, ou sous forme de granulés obtenus par les techniques connues de l'homme du métier telles que granulation par voie humide, en lit fluidisé, par atomisation, par extrusion, sphéronisation, compactage, spray-cooling, etc..

De bons résultats ont été obtenus avec des compositions filmogènes comprenant en poids :
- 10 à 15% en poids d'amidon de pois stabilisé
- 1 à 2% en poids de plastifiant
- éventuellement environ 5 à 7% d'opacifiant

De très bons résultats ont été obtenus avec des compositions filmogènes comprenant :
- 10 à 15% en poids d'amidon de pois hydroxypropylé
- 1 à 2% de glycérol
- 5 à 7% de talc
- le complément à 100% étant de l'eau et autres additifs tels que colorants et/ou arômes.

Dans le cas ou la composition selon l'invention se présente sous forme de poudre prête à l'emploi, la composition pulvérulente comprend avantageusement :
- de 15 à 75%, de préférence de 25 à 50% en poids d'amidon de pois stabilisé, de préférence hydroxypropylé, et encore plus préférentiellement hydroxypropylé et fluidifié,
- de 1 à 20% et de préférence de 5 à 12% en poids d'un agent filmogène secondaire,
- de 5 à 15% en poids d'un agent plastifiant,
- éventuellement 1 à 20% d'un promoteur d'adhésion comme notamment le lactose ou la cellulose microcristalline.

Selon une variante préférée de la présente invention, l'agent filmogène secondaire est un dérivé cellulosique et le plastifiant est le polyéthylène glycol ou le stéarate de polyéthylène glycol.

D'autres compositions adaptées à diverses applications sont décrites à titre illustratif dans les exemples qui suivent.

La composition filmogène conforme à l'invention est utilisée avantageusement pour le pelliculage de formes solides telles que : comprimés, gélules, capsules, pellets, granules, semences, de formes solides alimentaires telles que les biscuits, les céréales pour petit-déjeuner, les confiseries, pour préparer des capsules molles ou des gélules dures, ainsi que pour la fabrication de films destinés à toutes applications alimentaires, pharmaceutiques, agricoles, industrielles et autres. Elle peut également convenir avantageusement à l'enrobage de vitamines, de principes actifs notamment sous forme de poudres ou de cristaux. Elle peut jouer un rôle de protection de l'actif vis à vis de l'environnement (humidité, oxydation), de masquage de goût en particulier en présence de principes actifs amers ou présentant un goût désagréable, et peut également retarder la libération de l'actif en particulier en association avec un plastifiant hydrophobe. Dans le cas des semences, la composition filmogène leur confère un enrobage protecteur vis à vis de l'environnement et- en particulier vis à vis des attaques fongiques ou bactériennes.

Elle convient particulièrement bien pour la préparation de films contenant des principes actifs ou des films aromatiques encore appelés feuilles d'arôme. Il s'agit de feuilles très fines qui, placées sur la langue, fondent instantanément tout en diffusant un arôme, par exemple de menthe. Elle peut convenir également pour des applications pharmaceutiques ne nécessitant pas spécifiquement une fonte instantanée du film, comme la libération d'actifs éventuellement retardée par exemple ou la mucoadhésion.

Ainsi, une composition filmogène conforme à l'invention, présentant une viscosité inférieure ou égale à 500 mPa.s à 25°C et 10% de matière sèche permet de répondre au problème technique de la stabilité et processabilité aisée des compositions amylacées de l'art antérieur.

Les compositions d'amidons riches en amylose ou riches en amylopectine présentent toutes une viscosité telle en solution à 10% de matière sèche et 25°C qu'il est quasiment impossible de les utiliser dans les équipements usuels de pelliculage. C'est pourquoi la présente invention vise une composition amylacée filmogène apte au pelliculage de formes solides ou à la préparation de films, caractérisée en ce qu'elle présente une viscosité inférieure à 500 mPa.s à 25°C et 10% de matière sèche.

La viscosité au sens de la présente invention est une viscosité Brookfield déterminée au moyen par exemple d'un viscosimètre Brookfield RVF 100, en utilisant le mobile de l'appareil qui donne une lecture comprise entre 20 et 80% de l'échelle du cadran de l'appareil, à une vitesse de rotation de 100 tours par minute.

Selon une variante avantageuse, ladite composition amylacée présente une teneur en amylose comprise entre 25 et 45%, de préférence entre 30 et 44%, encore plus préférentiellement entre 35 et 40%, et mieux encore entre 35 et 38% en poids sec par rapport au poids sec d'amidon total.

La présente invention vise en outre un procédé de pelliculage de formes solides, caractérisé en ce qu'il comprend la pulvérisation d'une composition filmogène selon l'invention, sur un lit de noyaux en mouvement. La composition selon l'invention permet en effet de façon tout à fait avantageuse et nouvelle, une pulvérisation à température ambiante, c'est à dire de l'ordre de 20°C, ce que ne permettaient pas les compositions de l'art antérieur.

Elle concerne par ailleurs un procédé d'enrobage de formes solides caractérisé en ce qu'il comprend l'immersion des formes solides dans une composition filmogène selon l'invention, pour la réalisation de « caplets ».

Pour la réalisation du pelliculage de formes solides, on peut utiliser toute technique connue de l'homme du métier, telle que lit fluidisé, atomisation, pulvérisation, sphéronisation, en turbine de dragéification. A titre indicatif, on peut procéder comme suit : la composition filmogène est utilisée à une matière sèche de 10 à 30%, de préférence de 15 à 20%. Elle est éventuellement préalablement précuite à 90°C, et préchauffée à la température de pulvérisation souhaitée, généralement de l'ordre de 50 - 55°C. Le lit de comprimés est préchauffé à environ 55°C, et la composition filmogène est pulvérisée sur ce lit de noyaux en mouvement, en maintenant une température de l'ordre de 55°C.

Pour la fabrication de « caplets », l'on procède par exemple à l'immersion des comprimés mécaniquement ou à la main dans un bain contenant la composition filmogène selon l'invention.

Pour la fabrication de gélules, on peut utiliser un équipement conventionnel qui consiste à plonger des doigts métalliques dans la composition filmogène maintenue à température constante. Pour la préparation de capsules molles, on peut utiliser les techniques connues de formage sur tambours, ou par extrusion.

Pour la fabrication de films et en particulier de feuilles d'arômes, on procède par exemple par étalement d'une épaisseur faible et constante de la composition filmogène sur une surface plane ou cylindrique, suivi d'un séchage à -température ambiante ou à chaud. La matière sèche de la solution à enduire est choisie en fonction du temps de séchage que l'on souhaite appliquer. A titre indicatif, on choisit généralement une matière sèche comprise entre 50 et 90%.

L'on a obtenu des films de très bonne qualité comprenant en poids :
- 10 à 15% en poids d'amidon de pois hydroxypropylé prégélatinisé et/ou fluidifié,
- 1 à 3% de glycérol

L'invention sera mieux comprise à la lecture des exemples qui suivent, qui se veulent uniquement illustratifs et non limitatifs.

### Exemple 1 : PELLICULAGE DE COMPRIMES

On évalue différentes compositions de pelliculage de composition suivante :

**Tableau 1**

| **Composé** | **Fonction** | **Marque** | **Référence** |
|---|---|---|---|
| Amidon (cf. tableau 2) | Agent filmogène | Roquette | |
| Glycérol | Plastifiant | J.T Baker | 7044 |
| Talc poudre très fine | Opacifiant | Merck | Art. 8070 |
| Dioxyde de titane | Opacifiant | Prolabo | |
| Eurocert Indigo carmine | Colorant | Warner Jenkinson | 0036904 |
| Eau déminéralisée | Véhicule | | |

Les différents types d'amidon testés :

**Tableau 2**

| **Nature de l'amidon** | **Type** |
|---|---|
| Manioc, 20% amylose | Hydroxypropylé (degré de substitution DS = 0,2) |
| Pois, 35-39% amylose | Hydroxypropylé (DS = 0,2) |
| | Fluidifié acide Acétylé (DS = 0,021) |
| | Hydroxypropylé (DS = 0,2) fluidifié acide |
| Maïs waxy, 21% amylose | Prégélatinisé |
| Maïs riche en amylose (70%) | Prégélatinisé Hydroxypropylé |
| Mélange d'amidons waxy et riche en amylose, teneur en amylose du mélange 42% | Waxy natif et amidon riche en amylose hydroxypropylé (DS = 0,10) |

Les comprimés à pelliculer sont des comprimés concaves de 10mm de diamètre, 330mg de moyenne, de composition : 99% mannitol PEARLITOL^{®} 200SD et 1% de stéarate de magnésium.

On utilise le matériel suivant : bol double paroi en inox, mélangeur à pale IKA RW25W, homogénéisateur Ultra Turrax T25, pompe péristaltique, machine à enrober type lit d'air fluidisé, Glatt WSG 3V.

La solution d'enrobage à 20% de matière sèche comprend 12,5% d'amidon, 1,25% de plastifiant, 6,25% d'opacifiant (talc et dioxyde de titane), 80% d'eau, et une quantité négligeable de colorant.

La phase liquide est mélangée, l'amidon est ajouté à ce mélange, ensuite chauffé à 90°C. On ajoute le talc, et on maintient la solution sous agitation pendant 30 minutes à 90°C, puis elle est refroidie à la température de pulvérisation choisie.

Pour l'enrobage des comprimés, on préchauffe le lit de comprimés et l'équipement à 55°C. La pulvérisation de la solution filmogène est arrêtée quand 300 grammes ont été effectivement distribués.

La notation des formules se fait par comparaison entre les différentes formules testées avec +++ pour les meilleures performances et 0 pour les plus mauvaises.

Les viscosités mesurées sont des viscosités Brookfield, déterminées pour des solutions à 10% de matière sèche et à 25°C, au moyen par exemple d'un viscosimètre Brookfield RVF 100, en utilisant le mobile de l'appareil qui donne une lecture comprise entre 20 et 80% de l'échelle du cadran de l'appareil, à une vitesse de rotation de 100 tours par minute.

L'amidon waxy a généré du collage très rapidement et tout au long de l'enrobage, ainsi que la formation d'agglomérats. L'enrobage n'a donc pas pu se faire correctement, c'est pourquoi aucune caractérisation du film et de son aspect n'ont été faites.

La gélification a écarté de la sélection les solutions à base d'amidons riches en amylose.

La viscosité trop élevée de la solution à base d'amidon de manioc hydroxypropylé l'a écarté également car elle ne permet pas de pulvériser de manière correcte.

La solution à base d'amidon de pois hydroxypropylé ne rétrograde pas même à température ambiante pendant plus de 24 heures.

L'ensemble des résultats est repris dans le tableau ci-après :

**Tableau 3**

| **Amidon** | **Type** | **Viscosité à 25°C et 10% MS (mPa.s)** | **Solidité du film** | **Aspect lisse** | **Toucher non collant** | **Absence d'agglomérats lors enrobage** | **Aspect enrobage** |
|---|---|---|---|---|---|---|---|
| Amidon de pois (amylose 35/39%) | Hydroxypropylé (DS 0,2) | ++ (270) | +++ | ++ | +++ | +++ | +++ |
| | Hydroxypropylé (DS = 0,2) fluidifié | +++ (126) | +++ | +++ | +++ | +++ | +++ |
| | Fluidifié Acétylé (DS = 0, 021) | +++ (24) | + | +++ | +++ | ++ | + |
| Maïs riche en amylose (70%) | Prégel Hydroxypropylé (DS = 0,21) | 0 (>500) | +++ | ++ | +++ | + | +++ |
| Manioc, 20% amylose | Hydroxypropylé (DS = 0,2) | 0 (>500) | - | - | - | +++ | - |
| Maïs waxy, 21% amylose | Prégélatinisé | (421) | nd | - | - | 0 | nd |
| Mélange d'amidons à 42% d'amylose | Waxy natif Maïs riche en amylose hydroxypropylé (DS = 0,10) | ++ (246) | ++ | ++ | +++ | ++ | +++ |

Les amidons ou mélanges d'amidons présentant une teneur en amylose comprise entre 25 et 45% et au moins un amidon de légumineuse hydroxypropylé ou acétylé présentant une teneur en amylose inférieure à 45% montrent une nette supériorité pour une utilisation en tant qu'agent filmogène, par rapport aux amidons waxy ou aux amidons riches en amylose (plus de 45%). L'amidon de pois hydroxypropylé à un degré de substitution de 0,2 maximum éventuellement fluidifié donne les meilleurs résultats. Cette solution a pu être pulvérisée à froid sur les comprimés ce qui est particulièrement avantageux et donne un meilleur aspect des comprimés qu'à chaud.

### Exemple 2 : PREPARATION DE FILMS

On utilise le matériel suivant : bol double paroi en inox avec circulation interne d'huile pour le chauffage, mélangeur à pale IKA RW25W, mélangeur sous vide GUEDU type 4,5 NO, applicateur de film automatique.

Les solutions filmogènes testées ont la composition suivante :

| Quantités en grammes | FORMULE 1 | FORMULE 2 |
|---|---|---|
| Amidon de pois 37% amylose, hydroxypropylé (DS = 0,20) prégélatinisé | 67,5 | |
| Amidon de pois 37% amylose, hydroxypropylé (DS = 0,20) fluidifié | | 67,5 |
| Glycérol | 9,0 | |
| Arôme menthe | 7,5 | |
| Menthol | 7,5 | |
| Lécithine de soja | 0,5 | 0,75 |
| Saccharinate de sodium | 2,5 | |
| Colorant (solution 1%) | 1,5 | |
| eau | 404 | 403,75 |

On mélange dans le bol inox l'eau, la glycérine, le colorant et le saccharinate de sodium pendant 5 minutes. L'amidon est ensuite ajouté et dispersé pendant 5 minutes. Le mélange est chauffé à 70°C et maintenu à cette température pendant 10 minutes, puis transvasé dans le GUEDU préchauffé à 40°C et désaéré sous vide pendant 5 minutes. On solubilise ensuite le menthol dans l'arôme et la lécithine de soja, pour l'incorporer dans le GUEDU, le tout est mélangé sous vide pendant 10 minutes. La composition obtenue est étalée selon une épaisseur de 0,4 mm sur une plaque de plexiglas au moyen de l'applicateur automatique de films. Le film obtenu est séché à température ambiante puis découpé à la dimension souhaitée pour former des feuilles d'arôme.

Les feuilles d'arôme obtenues avec ces deux formules présentent une solidité et une rigidité adaptée à leur conditionnement et aux manipulations. La dissolution en bouche est rapide, et l'impact aromatique élevé leur confère une fonction de rafraîchisseur d'haleine.

Avec les compositions de l'art antérieur telles qu'à base d'amidon de maïs riche en amylose (70%), natifs ou modifiés, il est possible d'obtenir des feuilles d'arôme présentant une texture voisine, mais les températures de mise en oeuvre élevées indispensables pour éviter la rétrogradation de l'amidon et pour permettre l'étalement de la solution filmogène, crée une évaporation importante de l'arôme : les feuilles d'arôme ne présentent plus d'impact aromatique et n'ont plus la fonction « rafraîchissante » recherchée.

Avec les compositions de l'art antérieur à base d'amidons à teneur élevée en amylopectine (amidons waxy, plus de 75% d'amylopectine, moins de 25% d'amylose), les films ne présentent pas la cohésion nécessaire : ils se fissurent au séchage.

Seules les compositions filmogènes présentant une teneur en amylose comprise entre 25% et 45% permettent l'obtention de feuilles d'arôme présentant la texture et l'impact aromatique souhaités.

### Exemple 3 : pelliculage de comprimés vitaminés

Différentes compositions conformes à l'invention sont réalisées pour le pelliculage de comprimés vitaminés comme suit :

### Composition 1

| | |
|---|---|
| Amidon de pois hydroxypropylé : | 10,25% |
| Fluidifié | |
| Alginate | 0,6% |
| Lécithine | 0,38% |
| Triacétine | 0,31% |
| PEG 8000 | 0,94% |
| Laque | 1,87% |
| Dioxyde de titane | 0,6% |
| Eau | 85% |

### Composition 2

L'amidon de pois selon l'invention est combiné avec un dérivé cellulosique de manière à améliorer l'adhérence de la composition aux comprimés.

| | |
|---|---|
| Amidon de pois hydroxypropylé : | 5,15% |
| Fluidifié | |
| Hydroxypropyl méthylcellulose : | 5,15% |
| Alginate | 0,6% |
| Lécithine | 0,38% |
| Triacétine | 0,31% |
| PEG 8000 | 0,94% |
| Laque | 1,87% |
| Dioxyde de titane | 0,6% |
| Eau | 85% |

### Composition 3

| | |
|---|---|
| Amidon de pois hydroxypropylé : | 4,8% |
| Fluidifié | |
| Hydroxypropyl méthylcellulose : | 5,25% |
| Hydroxyéthyl cellulose | 1,5% |
| Dioxyde de titane | 2,25% |
| Acide stéarique éthoxylé | 1,2% |
| Dioxyde de titane | 2,25% |
| Eau | 85% |

Ces trois compositions sont pulvérisées sur des comprimés de vitamine C. Elles permettent d'obtenir un film brillant, d'excellente adhésion, et permettent de revêtir des comprimés gravés avec pas ou peu de remplissage du logo.

## Revendications

1. Composition amylacée filmogène pour le pelliculage de formes solides ou la préparation de films, **caractérisée en ce qu'**elle présente une teneur en amylose comprise entre 25 et 45%, ces pourcentages étant exprimés en poids sec par rapport au poids sec d'amidon contenu dans ladite composition, et **en ce qu'**elle comprend au moins un amidon de légumineuse hydroxypropylé ou acétylé présentant une teneur en amylose inférieure à 45%.

2. Composition selon la revendication 1, **caractérisée en ce qu'**elle présente une teneur en amylose comprise entre 30 et 44% ces pourcentages étant exprimés en poids sec par rapport au poids sec d'amidon contenu dans ladite composition

3. Composition selon revendications 1 ou 2, **caractérisée en ce que** ledit amidon de légumineuse est hydroxypropylé et fluidifié.

4. Composition selon l'une quelconque des revendication 1 à 3, **caractérisé en ce qu'**elle comprend en outre un plastifiant.

5. Composition selon la revendication 4, **caractérisée en ce que** ledit plastifiant est choisi dans le groupe constitué par le sorbitol, le glycérol, le polyéthylène glycol, le triéthylcitrate, le polysorbate, la cire de Carnauba, l'huile de ricin hydrogénée, seuls ou en mélange entre eux.

6. Composition selon l'une quelconque des revendications 1 à 5, **caractérisée en ce qu'**elle comprend en outre un agent filmogène secondaire choisi dans le groupe constitué par les dérivés cellulosiques, les alginates, les carraghénates, la polyvinylpyrrolidone, le phtalate de polyvinyle, le dextrose, la zéine, le pullulan, les polymères acryliques, le polyvinyl alcool, la gélatine, les dextrines, et leurs mélanges.

7. Composition selon l'une quelconque des revendications 1 à 6, **caractérisée en ce qu'**elle comprend 10 à 15% en poids d'amidon de pois hydroxypropylé ou acétylé et 1 à 2% en poids de plastifiant.

8. Composition selon l'une quelconque des revendications 1 à 6, **caractérisée en ce qu'**elle est pulvérulente et qu'elle comprend : de 15 à 75% en poids d'amidon de pois hydroxypropylé ou acétylé, ou hydroxypropylé et fluidifié, de 1 à 20% en poids d'un agent filmogène secondaire.

9. Composition selon la revendication 8 **caractérisée en ce qu'**elle comprend en outre de 1 à 20% de cellulose microcristalline.

10. Composition selon l'une ou l'autre des revendications 8 et 9 **caractérisée en ce qu'**elle comprend en outre de 5 à 15% de plastifiant en poids sec par rapport à ladite composition.

11. Composition selon l'une quelconque des revendications 8 à 10, **caractérisée en ce que** l'agent filmogène secondaire est un dérivé cellulosique.

12. Composition selon l'une quelconque des revendications 1 à 11, **caractérisée en ce qu'**elle présente une viscosité inférieure à 500 mPa. s à 25°C et 10% de matière sèche.

13. Composition selon l'une quelconque des revendications 1 à 12, **caractérisée en ce qu'**elle comprend au moins une substance active pharmaceutique.

14. Procédé de pelliculage de formes solides, **caractérisé en ce qu'**il comprend la pulvérisation d'une composition filmogène selon l'une quelconque des revendications 1 à 12 sur un lit de noyaux en mouvement.

15. Procédé d'enrobage de formes solides, **caractérisé en ce qu'**il comprend l'immersion des formes solides dans une composition filmogène selon l'une quelconque des revendications 1 à 13.

16. Procédé de préparation de films, **caractérisé en ce qu'**il consiste en l'étalement d'une composition filmogène selon l'une quelconque des revendications 1 à 13 sur une surface plane ou cylindrique, suivi d'un séchage à température ambiante ou à chaud.

17. Utilisation d'une composition filmogène selon l'une quelconque des revendications 1 à 13 pour la fabrication de films, capsules, gélules.

## Claims

1. Film-forming starchy composition for the film-coating of solid forms or for the preparation of films, **characterised in that** it has an amylose content between 25 and 45%, these percentages being expressed by dry weight with respect to the dry weight of starch present in said composition, and **in that** it comprises at least one hydroxypropylated or acetylated legume starch having an amylose content less than 45%.

2. Composition as claimed in Claim 1, **characterised in that** it has an amylose content between 30 and 44%, these percentages being expressed by dry weight with respect to the dry weight of starch present in said composition.

3. Composition as claimed in Claim 1 or 2, **characterised in that** said legume starch is hydroxypropylated and fluidification-treated.

4. Composition as claimed in any one of Claims 1 to 3, **characterised in that** it further comprises a plasticiser.

5. Composition as claimed in Claim 4, **characterised in that** said plasticiser is selected from the group composed of sorbitol, glycerol, polyethylene glycol, triethyl citrate, polysorbate, carnauba wax, hydrogenated castor oil, alone or as a mixture with one another.

6. Composition as claimed in any one of Claims 1 to 5, **characterised in that** it further comprises a secondary film-forming agent selected from the group composed of cellulose derivatives, alginates, carrageenates, polyvinylpyrrolidone, poly(vinyl phthalate), dextrose, zein, pullulan, acrylic polymers, polyvinyl alcohol, gelatine, dextrins, and mixtures thereof.

7. Composition as claimed in any one of Claims 1 to 6, **characterised in that** it comprises 10 to 15% by weight of hydroxypropylated or acetylated pea starch and 1 to 2% by weight of plasticiser.

8. Composition as claimed in any one of Claims 1 to 6, **characterised in that** it is pulverulent and that it comprises: 15 to 75% by weight of hydroxypropylated or acetylated, or hydroxypropylated and fluidification-treated, pea starch and 1 to 20% by weight of a secondary film-forming agent.

9. Composition as claimed in Claim 8, **characterised in that** it further comprises 1 to 20% of microcrystalline cellulose.

10. Composition as claimed in either one of Claims 8 and 9, **characterised in that** it further comprises 5 to 15% of plasticiser by dry weight with respect to said composition.

11. Composition as claimed in any one of Claims 8 to 10, **characterised in that** the secondary film-forming agent is a cellulose derivative.

12. Composition as claimed in any one of Claims 1 to 11, **characterised in that** it has a viscosity less than 500 mPa.s at 25°C and a solids content of 10%.

13. Composition as claimed in any one of Claims 1 to 12, **characterised in that** it comprises at least one pharmaceutically active substance.

14. Process for film-coating solid forms, **characterised in that** it comprises the spraying of a film-forming composition as claimed in any one of Claims 1 to 12 on a bed of moving cores.

15. Process for coating solid forms, **characterised in that** it comprises the immersion of the solid forms in a film-forming composition as claimed in any one of Claims 1 to 13.

16. Process for the preparation of films, **characterised in that** it consists in spreading a film-forming composition as claimed in any one of Claims 1 to 13 on a flat or cylindrical surface, followed by drying at ambient temperature or under hot conditions.

17. Use of a film-forming composition as claimed in any one of Claims 1 to 13 for the manufacture of films, capsules and hard gelatin capsules.

## Patentansprüche

1. Filmbildende stärkehaltige Zusammensetzung für die Beschichtung von festen Formen oder die Herstellung von Filmen, **dadurch gekennzeichnet, dass** sie einen Amylosegehalt aufweist, umfassend zwischen 25 und 45 %, wobei diese Prozentanteile als Trockengewicht bezogen auf das Trockengewicht von Stärke, die in der Zusammensetzung enthalten ist, angegeben sind, und dadurch, dass sie mindestens eine hydroxypropylierte oder acetylierte Hülsenfruchtstärke umfasst, die einen Amylosegehalt von unter 45 % aufweist.

2. Zusammensetzung nach Anspruch 1, **dadurch gekennzeichnet, dass** sie einen Amylosegehalt zwischen 30 und 44 % aufweist, wobei diese Prozentanteile als Trockengewicht bezogen auf das Trockengewicht von Stärke, die in der Zusammensetzung enthalten ist, angegeben sind.

3. Zusammensetzung nach Anspruch 1 oder 2, **dadurch gekennzeichnet, dass** die Hülsenfruchtstärke hydroxypropyliert und fluidifiziert ist.

4. Zusammensetzung nach einem der Ansprüche 1 bis 3, **dadurch gekennzeichnet, dass** sie weiterhin ein Plastifizierungsmittel enthält.

5. Zusammensetzung nach Anspruch 4, **dadurch gekennzeichnet, dass** das Plasifizierungsmittel ausgewählt wird aus der Gruppe, bestehend aus Sorbitol, Glycerol, Polyethylenglykol, Triethylcitrat, Polysorbat, Carnaubawachs, hydriertem Rizinusöl, einzeln oder im Gemisch miteinander.

6. Zusammensetzung nach einem der Ansprüche 1 bis 5, **dadurch gekennzeichnet, dass** sie weiterhin ein sekundäres filmbildendes Mittel umfasst, ausgewählt aus der Gruppe, bestehend aus Zellulosederivaten, Alginaten, Carrageenaten, Polyvinylpyrrolidon, Polyvinylphthalat, Dextrose, Zein, Pullulan, Acrylpolymeren, Polyvinylalkohol, Gelatine, Dextrinen und ihren Gemischen.

7. Zusammensetzung nach einem der Ansprüche 1 bis 6, **dadurch gekennzeichnet, dass** sie 10 bis 15 Gewichts -% hydroxypropylierte oder acetylierte Erbsenstärke und 1 bis 2 Gewichts -% Plastfizierungsmittel enthält.

8. Zusammensetzung nach einem der Ansprüche 1 bis 6, **dadurch gekennzeichnet, dass** sie pulverförmig ist und dass sie umfasst: von 15 bis 75 Gewichts -% hydroxypropylierte oder acetylierte, oder hydroxypropylierte und fluidifizierte Erbsenstärke, von 1 bis 20 Gewichts -% eines sekundären filmbildenden Mittels.

9. Zusammensetzung nach Anspruch 8, **dadurch gekennzeichnet, dass** sie weiterhin von 1 bis 20 % mikrokristalline Zellulose enthält.

10. Zusammensetzung nach einem der Ansprüche 8 oder 9, **dadurch gekennzeichnet, dass** sie weiterhin 5 bis 15 % Plastifizierungsmittel bezogen auf das Trockengewicht der Zusammensetzung enthält.

11. Zusammensetzung nach einem der Ansprüche 8 bis 10, **dadurch gekennzeichnet, dass** das sekundäre filmbildende Mittel ein Zellulosederivat ist.

12. Zusammensetzung nach einem der Ansprüche 1 bis 11, **dadurch gekennzeichnet, dass** sie eine Viskosität unter 500 mPa·s bei 25°C und 10 % Trockensubstanz aufweist.

13. Zusammensetzung nach einem der Ansprüche 1 bis 12, **dadurch gekennzeichnet, dass** sie mindestens eine pharmazeutisch wirksame Substanz aufweist.

14. Verfahren zur Beschichtung fester Formen, **dadurch gekennzeichnet, dass** es die Pulverisierung einer filmbildenden Zusammensetzung nach einem der Ansprüche 1 bis 12 über einem bewegten Kernbett umfasst.

15. Verfahren zur Ummantelung von festen Formen, **dadurch gekennzeichnet, dass** es das Eintauchen von festen Formen in eine filmbildende Zusammensetzung nach einem der Ansprüche 1 bis 13 umfasst.

16. Verfahren zur Herstellung von Filmen, **dadurch gekennzeichnet, dass** es aus der Verteilung einer filmbildenden Zusammensetzung nach einem der Ansprüche 1 bis 13 auf einer ebenen oder zylindrischen Oberfläche, gefolgt von einer Trocknung bei Raumtemperatur oder unter Wärme, besteht.

17. Verwendung einer filmbildenden Zusammensetzung nach einem der Ansprüche 1 bis 13 zur Herstellung von Filmen, Kapseln, Gelatinekapseln.
